# EUROPEAN PATENT SPECIFICATION

(11) **EP 2 900 116 B1**
(45) Date of publication and mention of the grant of the patent: **13.03.2019**
(21) Application number: 13841350.5
(22) Date of filing: 26.09.2013
(51) Int. Cl.: A47K 7/00, A61K 8/86, A61Q 19/00, A61K 8/02, A61K 8/41

(54) **WET WIPE CHEMICAL SOLUTION AND WET WIPE**
CHEMISCHE LÖSUNG FÜR EIN FEUCHTWISCHTUCH UND FEUCHTWISCHTUCH
SOLUTION DE PRODUIT CHIMIQUE POUR UNE LINGETTE HUMIDE ET LINGETTE HUMIDE

(30) Priority: 28.09.2012 JP 2012216969
(43) Date of publication of application: 05.08.2015
(73) Proprietor: Unicharm Corporation, Ehime 799-0111 (JP)
(72) Inventor: Ueda, Takahiro, Kanonji-shi Kagawa 769-1602 (JP); Kondoh, Nami, Kanonji-shi Kagawa 769-1602 (JP); Bandoh, Takeshi, Kanonji-shi Kagawa 769-1602 (JP)
(74) Representative: Dolleymores
(86) International application number: PCT/JP2013/005749
(87) International publication number: WO 2014/050128

(56) References cited:
- JP-A- 2004 059 486
- JP-A- 2004 529 148
- JP-A- 2005 255 213
- JP-A- 2005 296 240
- US-A1- 2005 008 680
- Tadashi &to ET AL: "Measurements of Light Scattering Intensities on Extremely Dilute Solutions of Nonionic Surfactant", Langmuir, 1 January 1995 (1995-01-01), pages 1867-1869, XP055237758, Retrieved from the Internet: URL:http://pubs.acs.org/doi/pdf/10.1021/la 00006a009

## Description

### TECHNICAL FIELD

The present invention relates to a chemical solution for a wet wipe and to a wet wipe. More specifically, the present invention relates to a paraben-free chemical solution for wet wipe and to a wet wipe impregnated with said solution.

### Background Art

Conventionally, a chemical solution for wet wipes contains a preservative for an antiseptic effect.

US 2005/0008680 A1 discloses wet wipes impregnated with a lotion composition where the lotion includes a preservative system and soothing ingredients to mitigate stinging.

Esters of paraoxybenzoic acid (hereinafter referred to as "paraben") have been widely used as the preservative.

However, paraben is designated by Japanese drug legislation as a substance that may cause skin irritation such as allergic reactions, and labeling thereof is mandatory when used. Given this, in recent years there has been progress in the development of a paraben-free wet wipe.

Since a wet wipe is used in direct contact with the human body, from the viewpoint of effects on the human body, it is preferable that the content of water, which is the main component of the chemical solution for wet wipe, is high. However, if paraben is omitted and the water content is increased accordingly, to replace paraben, hydrogen bonding caused by water increases adhesive strength of wet wipe base materials with respect to each other. Upon extracting the wet wipe from a package holding wet wipe base materials in layered units, a phenomenon occurs whereby a plurality of wet wipes successively slip out from the package, hindering ease of extraction of the wet wipes.

The present invention has been made in view of the abovementioned problem, and is aimed at providing a chemical solution for a wet wipe and a wet wipe that are paraben-free and for realizing ease of extraction thereof even with higher water content than conventional products.

### Means for Solving the Problems

The present invention provides the paraben-free chemical solution of independent claim 1 and the wet wipe composed of a layered body of claim 6. The dependent claims specify preferred but optional features.

The present invention provides a paraben-free chemical solution for wet wipe in which the product of chemical solution film thickness, measured by a measurement method specified below, and chemical solution surface tension, is at least 810 um.mN/m. Preferably, the value of said product is up to 1020 um.mN/m.

### Measurement Method for Liquid Film Thickness of Chemical Solution

Drop 0.03 ml of the chemical solution on a glass slide measuring 26 mm by 90 mm, and 1 mm thick with a pipette, and gently place another glass slide of the same size thereon without the application of pressure. Then, allow the glass slides to stand for 30 seconds, and measure the chemical solution film thickness interposed between the glass slides by magnified observation with a microscope from a position immediately lateral to the glass slides.

In this application "um" shows micrometer.

The present invention also provides a wet wipe composed of a layered body of a wet wipe base material, wherein the wet wipe is impregnated with the chemical solution for wet wipe; and a wet wipe composed of a wet wipe base material which is impregnated with the chemical solution for a wet wipe.

### Summary of Invention

According to the present invention, it is possible to provide a chemical solution for wet wipe and a wet wipe that are paraben-free and facilitate ease of extraction thereof even with higher water content than conventional products.

### Brief Description of Drawings

FIG. 1 is a partially cutaway perspective view of a wet wipe package that houses a wet wipe according to one embodiment of the present invention;
FIG. 2 is a perspective view of wet wipe base material that is V-Z folded; and
FIG. 3 is a graph showing a relationship between: the product of chemical solution film thickness and chemical solution surface tension, and ease of extraction of the wet wipe.

### Description of Embodiments

A preferred embodiment of the present invention is described in detail below.

First, a chemical solution for wet wipe according to the present embodiment is described.

The chemical solution for wet wipe according to the present embodiment is a so-called paraben-free chemical solution that does not contain paraben, which has conventionally been widely used as a preservative. A wet wipe base material is impregnated with the chemical solution for wet wipe according to the present embodiment and made into a layered body, thereby obtaining a wet wipe 10 described later.

The product of chemical solution film thickness in the present embodiment, measured by a measurement method described later, and chemical solution surface tension is at least 810 um.mN/m. This facilitates extraction of wet wipes. In the present embodiment, the product of the chemical solution film thickness as measured by the measurement method described herein, and the chemical solution surface tension is up to 1020 um.mN/m.

As a method for achieving a value for the product of the chemical solution film thickness and the chemical solution surface tension of at least 810 um.mN/m, a method of including in the chemical solution a non-ionic surface active agent in an amount of 0.001 - 0.003% by mass of the solution is used, wherein the non-ionic surface active agent is selected from a polyoxyethylene alkyl ether (described later).

Here, if the liquid film thickness of the chemical solution with which the wet wipe base material is impregnated is small, it is considered that the distance between the sheets of the base material that are layered will be small, the number of separated points will be reduced, and adhesive strength therebetween will increase.

In addition, high surface tension of the chemical solution with which the wet wipe base material is impregnated indicates high water content in the chemical solution. Here, if the surface tension of the chemical solution with which the wet wipe base material is impregnated is high, it is considered that adhesive strength between sheets of the wet wipe base material that are layered will increase due to hydrogen bonding caused by water.

Given this, a correlation can be found between the product of chemical solution film thickness and chemical solution surface tension, and ease of extraction of the wet wipe. The present embodiment improves ease of extraction of the wet wipe by setting the product to be within a predetermined range (at least 810 um.mN/m and up to 1020 um.mN/m in the present embodiment).

While it is difficult to measure the chemical solution film thickness for the wet wipe base material, the applicants have found that the chemical solution film thickness on a glass slide and the chemical solution film thickness on the surface of a wet wipe base material such as nonwoven fabric tend to be similar to each other.

Given this, in the present embodiment the chemical solution film thickness is measured by the following method.

Drop 0.03 ml of the chemical solution on a glass slide measuring 26 mm by 90 mm, and 1 mm thick with a pipette, and gently place another glass slide of the same size thereon without the application of pressure. Then, allow the glass slides to stand for 30 seconds, and measure the chemical solution film thickness interposed between the glass slides by magnified observation with a microscope from a position immediately lateral to the glass slides.

A digital microscope VHX (registered trademark) -2000 manufactured by KEYENCE CORPORATION may be used.

In the present embodiment the value of the surface tension of the chemical solution is measured by the following method.

First, pour 3 ml of the chemical solution into a disposable sample cup made of resin. Next, attach the sample cup to a surface tentiometer installed at a horizontal and vibration free location. Then dip a probe of the surface tentiometer into the chemical solution in the sample cup to measure the surface tension.

A multi-function high-speed surface tentiometer EZ-Pi Plus (distributed by Keystone Scientific K.K.) may be used.

The chemical solution for wet wipe according to the present embodiment contains water (pure water) as its main component. The water content is at least 99% by mass.

Here, since the main component of the chemical solution for wet wipe is water, it has been necessary to include a solubilizing agent (for example, propylene glycol (PG)) that solubilizes paraben, which is insoluble in water, when paraben is included in the chemical solution as in conventional products. However if the chemical solution is made to be paraben free, it is no longer necessary to include the solubilizing agent and the water content of the chemical solution can be increased instead. However, if the content of water in the chemical solution is increased, hydrogen bonding caused by the water increases the adhesive strength of the wet wipe base materials such as nonwoven fabric with respect to each other, leading to a deterioration in ease of extraction.

On the other hand, the present embodiment can provide a wet wipe that can be easily extracted even with high water content of at least 99% by mass, which is higher than that of conventional products (generally 97 to 98% by mass), by setting the product of the chemical solution film thickness and the chemical solution surface tension to be at least 810 um.mN/m, or by setting it to be in the range of 810 - 1020 um.mN/m.

The chemical solution for wet wipe according to the present embodiment includes a preservative selected from benzoic acid, iodopropynyl butylcarbamate (hereinafter referred to as IPBC), polyaminopropyl biguanide (hereinafter referred to as CQ, since COSMOCIL CQ (registered trademark), manufactured by Nikko Chemicals Co., Ltd., is used, for example), ethylenediaminetetraacetic acid disodium salt dihydrate (hereinafter referred to as EDTA-2Na), benzalkonium chloride, instead of paraben. The preservatives can be used either singly or in combination. A desired preservative effect can thus be obtained.

It should be noted that total preservative content is lower than 1%, more preferably lower than 0.2%. The water content is thus at least 99% by mass.

In addition, the chemical solution for wet wipe according to the present embodiment contains 0.001 to 0.003% by mass of a non-ionic surface active agent. The inclusion of a non-ionic surface active agent within this range is one means of achieving a value for the product of the chemical solution film thickness and the chemical solution surface tension of at least 810 um.mN/m, or in the range of 810 - 1020 um.mN/m.

As the non-ionic surface active agent, polyoxyethylene alkyl ethers are used, and among these, polyoxyethylene sec-tridecyl ether is preferably used. In addition, a commercially available product can also be used as the non-ionic surface active agent. For example, NIKKOL (registered trademark) BT-12 (manufactured by Nikko Chemicals Co., Ltd.), which is a commercially available polyoxyethylene sec-tridecyl ether, can be used.

The chemical solution for wet wipe according to the present embodiment having the above described configuration is prepared by, for example, the following method.

For preparing the chemical solution for wet wipe containing benzoic acid, IPBC, CQ, EDTA-2Na and benzalkonium chloride as preservatives: first, predetermined amounts of CQ, EDTA-2Na and benzalkonium chloride are dissolved in pure water. Then, predetermined amounts of benzoic acid and IPBC are dissolved in PG as the solubilizing agent, and a predetermined amount of a mixture thus obtained is added to the pure water solution of CQ, EDTA-2Na and benzalkonium chloride, agitated, and dissolved. The chemical solution for wet wipe according to the present embodiment is thus obtained.

It should be noted that CQ and benzalkonium chloride can be first dissolved in PG and then added to pure water.

By impregnating the wet wipe base material with the chemical solution for wet wipe according to the present embodiment thus prepared, the wet wipe is obtained. The impregnation of the chemical solution for wet wipe is carried out, for example, by spraying the chemical solution for wet wipe onto the wet wipe. The impregnation rate of the chemical solution for wet wipe is set to be 200 to 300% with respect to the wet wipe base material, from the viewpoint of facilitating wiping. The impregnation rate of the chemical solution within this range can sufficiently realize an effect of the present embodiment to facilitate wipe extraction.

The above specified impregnation rate is the mass of the chemical solution with respect to the mass of the wet wipe base material. For example, given a mass of the wet wipe base material of 100 mass parts and a mass of the chemical solution of 250 mass parts, the impregnation rate is 250%.

As the wet wipe base material, for example, a nonwoven fabric composed of natural fibers or synthetic fibers, paper, or the like, can be used. More specifically, a spun-lace nonwoven fabric of high wet strength obtained by combining a hydrophilic fiber such as rayon and pulp with at least one synthetic fiber, such as polyesther, polyethylene, or polypropylene, is used.

The size of the wet wipe base material is appropriately set according to usage and a container or package for holding the wipe base material. In the present embodiment, the size is set to be 200 mm by 150 mm, for example.

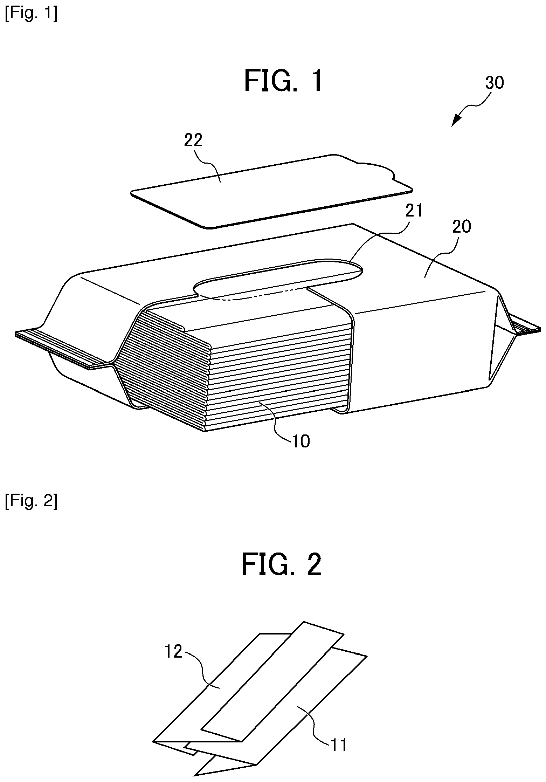

As a method of folding the wet wipe base material, for example, a Z fold (folding to have a cross-section orthogonal to the folding lines in a Z-shape), a modified Z fold (folding to have a cross-section orthogonal to the folding lines in a Z-shape, with an upper flap being longer than a lower flap), a V-Z fold (alternately layering a base material with a cross section orthogonal to the folding lines in a V-shape and a base material with a cross section orthogonal to the folding lines in a Z-shape), and the like, are employed. FIG. 2 is a perspective view of the wet wipe base material that is V-Z folded. As shown in FIG. 2, the V-Z fold layers a base material 11 folded with a V-shaped cross section and a base material 12 folded with a Z-shaped cross section alternately.

In the present embodiment, any of the methods of folding can realize ease of extraction.

Next, the wet wipe 10 according to the present embodiment is described.

FIG. 1 is a partially cutaway perspective view of a wet wipe package 30 that houses the wet wipe 10 according to the present embodiment.

As illustrated in FIG. 1, the wet wipe 10 is composed of a layered body in which a plurality of sheets of the wet wipe base material impregnated with the above described chemical solution for wet wipe are respectively folded at predetermined folding lines and then layered.

The wet wipe 10 is housed in a pillow-shaped package 20 having a label member 22 as a lid member and an opening 21, thereby composing the wet wipe package 30. The wet wipe 10 housed in the package 20 is extracted sheet by sheet from the opening 21 to be used. The wet wipe 10 is extracted from the opening by a user pinching a side edge portion thereof located on the opening 21 side.

An embodiment of the present invention has been explained heretofore; however, the present invention is not limited thereto and can be modified appropriately.

### EXAMPLES

The present invention is described more in detail hereinafter based on Examples, but the invention is not limited thereto.

### Examples 1 to 3 and Comparative Examples 1 to 3

As a paraben-free chemical solution for wet wipe, chemical solutions for wet wipe of Examples 1 to 3 and Comparative Example 1 to 3 were prepared according to compositions shown in Table 1. A method for preparing the solutions was as described above in the embodiment.

Sheets of wet wipe base material which were composed of nonwoven fabric (in this particular embodiment, the nonwoven fabric was composed of a spun-lace nonwoven fabric using rayon and/or pulp as a hydrophilic fiber and a synthetic fiber such as polypropylene and polyethylene as a hydrophobic fiber) and 200 mm by 150 mm in size were respectively impregnated with the chemical solutions thus prepared. The impregnation rate of the chemical solutions was 250%. Impregnation of the chemical solution for wet wipe was carried out by spraying the chemical solution for wet wipe onto the wet wipe.

Next, the sheets of wet wipe base material impregnated with the chemical solutions were V-Z folded as illustrated in FIG. 2, and layered to form a layered body, thereby obtaining wet wipes of Examples 1 to 3 and Comparative Example 1 to 3. The wet wipes thus obtained were housed in the packages as illustrated in FIG. 1 to thereby obtain wet wipe packages. The wet wipes were housed in such an orientation that the dimension thereof in the direction of extraction from the opening of the package is 200 mm.

### Evaluation

The chemical solutions for wet wipe and wet wipes of the Examples and Comparative Examples were evaluated as follows.

### Successive Extraction Count

For each of the wet wipe packages obtained in the Examples and Comparative Examples, 80 plies of the wet wipe base material were extracted one by one from the opening of the package and the number of times a subsequent ply of the wet wipe base material successively slips out completely from the opening was counted as the successive extraction count. The successive extraction count was obtained by averaging the counts taken with n = 2 (i.e. 80 plies by 2, 160 plies). Results are shown in Table 1.

### Average Protruding Height

For each wet wipe package obtained in the Examples and Comparative Examples, 80 plies of the wet wipe base material were extracted one by one from the opening of the package and the height (mm) of a subsequent ply of the wet wipe base material that slips out in succession and is protruding from the opening was measured. The average protruding height was obtained by averaging the counts taken with n = 2 (i.e. 80 plies by 2, 160 plies). The protruding height of the subsequent ply of the wet wipe base material that has completely slipped out from the opening was defined as 200 mm, since the dimension thereof in the direction of extraction is 200 mm. Results are shown in Table 1.

### Liquid Film Thickness of Chemical Solution

The chemical solution film thickness for a wet wipe obtained in the Examples and Comparative Examples was measured as follows.

First, 0.03 ml of the chemical solution was dropped on a glass slide (26 mm by 90 mm, 1 mm in thickness) and then another glass slide of the same size was gently placed thereon. Next, the glass slides were allowed to stand for 30 seconds, and the chemical solution film thickness interposed between the glass slides was measured by magnified observation with a digital microscope VHX (registered trademark)-2000 (manufactured by KEYENCE CORPORATION) from a position immediately lateral to the glass slides. Results are shown in Table 1.

### Surface Tension of Chemical Solution

The surface tension of the chemical solutions of the Examples and Comparative Examples was measured by the following method.

First, 3 ml of the chemical solution was poured into a disposable sample cup made of resin. Next, the sample cup was attached to a multi-function high-speed surface tentiometer EZ-Pi Plus (distributed by Keystone Scientific K.K.) installed at a horizontal and vibration free location. Then, a probe of the surface tentiometer was dipped into the chemical solution in the sample cup to measure the surface tension. Results are shown in Table 1.

**[Table 1]**

| | Example | | | Comparative Example | | |
|---|---|---|---|---|---|---|
| | 1 | 2 | 3 | 1 | 2 | 3 |
| PG (MASS %) | 0.7 | 0.7 | 0.7 | 0.7 | 0.7 | 0.7 |
| METHYL PARABEN (MASS %) | 0 | 0 | 0 | 0 | 0 | 0 |
| ETHYL PARABEN (MASS %) | 0 | 0 | 0 | 0 | 0 | 0 |
| PROPYL PARABEN (MASS %) | 0 | 0 | 0 | 0 | 0 | 0 |
| CETYLPYRIDINIUM CHLORIDE (MASS %) | 0 | 0 | 0 | 0 | 0 | 0 |
| BENZOIC ACID (MASS %) | 0.1 | 0.1 | 0.1 | 0.1 | 0.1 | 0.1 |
| IPBC (MASS %) | 0.0075 | 0.0075 | 0.0075 | 0.0075 | 0.0075 | 0.0075 |
| CQ (MASS %) | 0.07 | 0.1 | 0.1 | 0.1 | 0.1 | 0.1 |
| EDTA-2Na (MASS %) | 0.05 | 0.05 | 0.05 | 0.05 | 0.05 | 0.05 |
| BENZALKONIUM CHLORIDE (MASS %) | 0.01 | 0.01 | 0.01 | 0.01 | 0.01 | 0.01 |
| NTKKOL BT-12 (MASS %) | 0.001 | 0.002 | 0.003 | 0 | 0.005 | 0.01 |
| PURE WATER (MASS %) | 99.0615 | 99.0305 | 99.0295 | 99.0325 | 99.0275 | 99.0225 |
| SUCCESSIVE EXTRACTION COUNT | 4 | 3.5 | 5 | 8.5 | 18.5 | 25.5 |
| AVERAGE PROTRUDING HEIGHT(mm) | 111.8 | 105.4 | 114.8 | 115.4 | 140.6 | 148.3 |
| SURFACE TENSION(mN/m) | 35.13 | 34.1 | 33.5 | 36.45 | 33.2 | 32.77 |
| CHEMICAL SOLUTION FILM THICKNESS(*µ*m) | 25.03 | 29.9 | 24.63 | 22.19 | 14.84 | 12.9 |
| CHEMICAL SOLUTION FILM THICKNESS × SURFACE TENSION(*µ*m·mN/m) | 879 | 1020 | 825 | 809 | 493 | 423 |

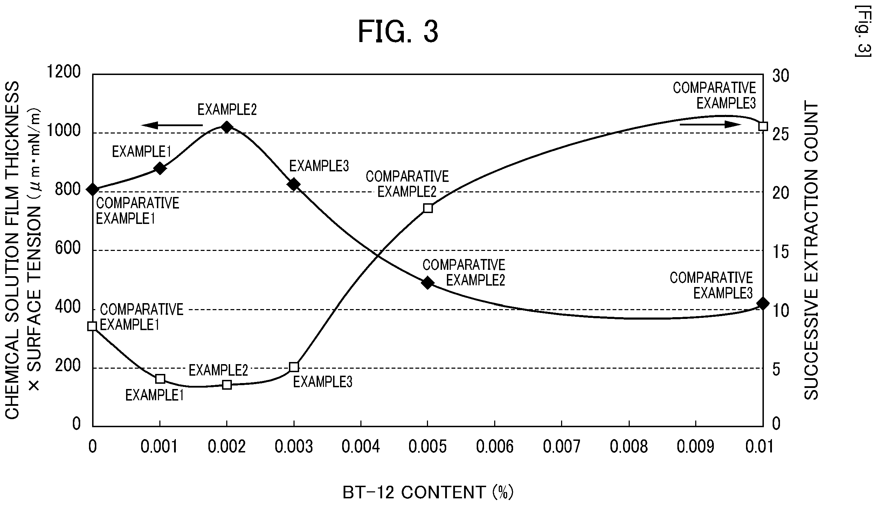

In addition, a relationship between: the product of the chemical solution film thickness and the chemical solution surface tension, and ease of extraction of the wet wipe, based on the result of evaluation of the Examples and Comparative Examples, is shown in FIG. 3.

As shown in Table 1 and FIG. 3, Examples 1 to 3, in which the product of the film thickness and the surface tension of the chemical solution is at least 810 um.mN/m, or in the range of 810 - 1020 um.mN/m, show low average protruding heights and successive extraction counts of 5 times or less; preferable extraction thereof is thus confirmed.

On the other hand, Comparative Examples 1 to 3, in which the product of the film thickness and the surface tension of the chemical solution is less than 810 um.mN/m, show higher average protruding heights than Examples 1 to 3 and high successive extraction counts; non-preferable extraction thereof is thus confirmed.

In addition, from the results of Examples 1 to 3, it was confirmed that the product of the film thickness and the surface tension of the chemical solution can be made at least 810 um.mN/m, even with a water content of at least 99% by mass due to the absence of paraben, by including 0.001 to 0.003% by mass of polyoxyethylene sec-tridecyl ether (NIKKOL (registered trademark) BT-12 manufactured by Nikko Chemicals Co., Ltd.) as the non-ionic surface active agent.

Thus a solution according to the present invention may comprise:
water in an amount of at least 99% by mass;
a non-ionic surface active agent in an amount of 0.001-0.003% by mass;
a preservative in an amount of up to 0.299% by mass; and
a solubilising agent for the preservative, in an amount or approximately 0.7% by mass.

The non-ionic surface active agent is a polyoxyethylene alkyl ether, preferably polyoxyethylene sec-tridecyl ether, and more preferably Nikkol BT-12 (registered trademark).

The preservative is selected from one or more of benzoic acid; iodopropynyl butyl carbamate; polyaminopropyl biguanide; ethylene diaminetetraacetic acid disodium salt dehydrate; and benzalkonium chloride.

The preservative is suitably propylene glycol.
The present disclosure includes:
(1) A paraben-free chemical solution for a wet wipe comprising water and a preservative, wherein the value of the product of the film thickness of the chemical solution and the surface tension of the chemical solution, is at least 810 um.mN/m and may include one or a combination of the following features:
(2) The value of the product of the film thickness of the chemical solution and the surface tension of the chemical solution, is up to 1020 um.mN/m.
(3) The solution contains a non-ionic surface-active agent selected from a polyoxyethylene alkyl ether.
(4) The polyoxyethylene alkyl ether is polyoxyethylene sec-tridecyl ether.
(5) The polyoxyethylene sec-tridecyl ether is Nikkol BT-12 (registered trademark).
(6) The preservative is selected from benzoic acid; iodopropynyl butyl carbamate; polyaminopropyl biguanide; ethylene diaminetetraacetic acid disodium salt dehydrate; benzalkonium chloride; and a mixture of two or more thereof.
(7) The solution containsa solubilizer for one or more of the preservatives.
(8) The solubilizer is propylene glycol.
(9) The film thickness of the chemical solution is as measured by the following method:
   chemical solution (0.03 ml) is dropped on to a glass slide measuring 26 mm by 90 mm, and 1 mm thick with a pipette,. Another glass slide of the same size is placed gently thereon. Then, allow the glass slides to stand for 30 seconds, after which the thickness of the film of chemical solution interposed between the glass slides is measured by magnified observation using a microscope from a position immediately lateral to the glass slides.
The present disclosure includes:
(10) A paraben-free chemical solution containing water in an amount of at least 99% by mass; and a non-ionic surface active agent in an amount of 0.001-0.003% by mass and optionally:
(11) Further containing a preservative in an amount of up to 0.299% by mass.
(12) Further comprising a solubilising agent for the preservative, in an amount or approximately 0.7% by mass.

Although the present invention has been described with reference to particular means, materials and embodiments, from the foregoing description, one skilled in the art can easily ascertain the essential characteristics of the present invention and various changes and modifications can be made to adapt the various uses and characteristics.

### Reference Sign List

- 10, 11, 12: Wet wipe(s)
- 20: Package
- 21: Opening
- 22: Label / Lid member
- 30: Wet wipe package

## Claims

1. A paraben-free chemical solution for wet wipe, the chemical solution containing water, a non-ionic surface active agent and a preservative, wherein
the non-ionic surface-active agent is selected from a polyoxyethylene alkyl ether;
the preservative is selected from benzoic acid; iodopropynyl butyl carbamate; polyaminopropyl biguanide; ethylene diaminetetraacetic acid disodium salt dehydrate; benzalkonium chloride; and a mixture of two or more thereof;
the chemical solution additionally contains a solubilizer for one or more of the preservatives;
the chemical solution contains water in an amount of at least 99% by mass and the non-ionic surface active agent in an amount of 0.001-0.003% by mass;
the product of chemical solution film thickness and chemical solution surface tension is at least 810 um.mN/m and
wherein the film thickness of the chemical solution is measured by:
dropping 0.03 ml of the chemical solution on a glass slide measuring 26 mm by 90 mm, and 1 mm thick with a pipette,
gently placing another glass slide of the same size thereon without the application of pressure,
allowing the glass slides to stand for 30 seconds, and
measuring the chemical solution film thickness interposed between the glass slides by magnified observation with a microscope from a position immediately lateral to the glass slides.

2. A paraben-free chemical solution according to claim 1, wherein the value of the product of the film thickness of the chemical solution and the surface tension of the chemical solution, is up to 1020 um.mN/m.

3. A paraben-free chemical solution according to claim 1 or 2, wherein the polyoxyethylene alkyl ether is polyoxyethylene sec-tridecyl ether.

4. A paraben-free chemical solution for a wet wipe according to any one of claims 1 to 3, further containing a preservative in an amount of up to 0.299% by mass.

5. A paraben-free chemical solution for a wet wipe according to claim 4, further comprising a solubilising agent for the preservative, in an amount or approximately 0.7% by mass.

6. A wet wipe (10) composed of a layered body of a wet wipe base material (11, 12), wherein the wet wipe is impregnated with the chemical solution for wet wipe according to any one of claims 1 to 5.

## Patentansprüche

1. Parabenfreie chemische Lösung für ein Feuchttuch, wobei die chemische Lösung Wasser, ein nichtionisches oberflächenaktives Mittel und ein Konservierungsmittel enthält, wobei
das nichtionische oberflächenaktive Mittel aus einem Polyoxyethylenalkylether ausgewählt ist;
das Konservierungsmittel aus Folgenden ausgewählt ist: Benzoesäure, Iodpropinylbutylcarbamat, Polyaminopropylbiguanid, Ethylendiamintetraessigsäure-Dinatriumsalz-Dihydrat, Benzalkoniumchlorid und einer Mischung aus zwei oder mehr davon;
die chemische Lösung zusätzlich einen Lösungsvermittler für eines oder mehrere der Konservierungsmittel enthält;
die chemische Lösung Wasser in einer Menge von mindestens 99 Masse-% und das nichtionische oberflächenaktive Mittel in einer Menge von 0,001-0,003 Masse-% enthält;
das Produkt von Filmdicke der chemischen Lösung und Oberflächenspannung der chemischen Lösung mindestens 810 um.mN/m beträgt und
wobei die Filmdicke der chemischen Lösung durch Folgendes gemessen wird:
Tropfen von 0,03 ml der chemischen Lösung auf einen Glasträger, der 26 mm mal 90 mm und 1 mm Dicke misst, mit einer Pipette,
vorsichtiges Legen eines weiteren Glasträgers der gleichen Größe darauf ohne Anwendung von Druck,
Ermöglichen, dass die Glasträger für 30 Sekunden liegen bleiben, und
Messen der Filmdicke der chemischen Lösung, die zwischen die Glasträger eingefügt wurde, durch vergrößerte Beobachtung mit einem Mikroskop von einer Position unmittelbar seitlich von den Glasträgern.

2. Parabenfreie chemische Lösung nach Anspruch 1, wobei der Wert des Produkts der Filmdicke der chemischen Lösung und der Oberflächenspannung der chemischen Lösung bis zu 1020 um.mN/m beträgt.

3. Parabenfreie chemische Lösung nach Anspruch 1 oder 2, wobei der Polyoxyethylenalkylether Polyoxyethylen-sec-tridecylether ist.

4. Parabenfreie chemische Lösung für ein Feuchttuch nach einem der Ansprüche 1 bis 3, die weiter ein Konservierungsmittel in einer Menge von bis zu 0,299 Masse-% enthält.

5. Parabenfreie chemische Lösung für ein Feuchttuch nach Anspruch 4, die weiter einen Lösungsvermittler für das Konservierungsmittel in einer Menge von ungefähr 0,7 Masse-% umfasst.

6. Feuchttuch (10) das aus einem geschichteten Körper eines Feuchttuchgrundmaterials (11, 12) besteht, wobei das Feuchttuch mit der chemischen Lösung für ein Feuchttuch nach einem der Ansprüche 1 bis 5 getränkt ist.

## Revendications

1. Solution chimique exempte de parabènes pour lingette humide, la solution chimique contenant de l'eau, un agent tensio-actif non ionique et un agent conservateur, où
l'agent tensio-actif non ionique est sélectionné parmi des alkyléthers de polyoxyéthylène ;
l'agent conservateur est sélectionné parmi l'acide benzoïque ; l'iodopropynyl-butylcarbamate ; le polyaminopropyl-biguanide ; le sel disodique déshydraté de l'acide éthylène-diamine-tétraacétique ; le chlorure de benzalkonium ; et un mélange de deux ou plusieurs de ceux-ci ;
la solution chimique contient en outre un agent de solubilisation de un ou plusieurs des agents conservateurs ;
la solution chimique contient de l'eau à une quantité de 99 % en masse au moins et l'agent tensio-actif non ionique à une quantité de 0,001-0,003 % en masse ;
le produit de l'épaisseur d'un film de la solution chimique et de la tension superficielle de la solution chimique est de 810 µm.mN/m au moins et
où l'épaisseur d'un film de la solution chimique est mesurée en :
déposant à la pipette 0,03 ml de la solution chimique sur une lame de verre mesurant 26 mm par 90 mm et d'une épaisseur de 1 mm,
plaçant délicatement sur celle-ci une autre lame de verre de mêmes dimensions sans appliquer une pression,
laissant les lames de verre reposer pendant 30 secondes et
mesurant l'épaisseur du film de la solution chimique interposé entre les lames de verre par observation grossie à l'aide d'un microscope à une position immédiatement latérale par rapport aux lames de verre.

2. Solution chimique exempte de parabènes selon la revendication 1, où la valeur du produit de l'épaisseur du film de la solution chimique et de la tension superficielle de la solution chimique va jusqu'à 1 020 µm.mN/m.

3. Solution chimique exempte de parabènes selon la revendication 1 ou 2, où l'alkyléther de polyoxyéthylène est le sec-tridécyléther de polyoxyéthylène.

4. Solution chimique exempte de parabènes pour lingette humide selon l'une quelconque des revendications 1 à 3 contenant en outre un agent conservateur à une quantité allant jusqu'à 0,299 % en masse.

5. Solution chimique exempte de parabènes pour lingette humide selon la revendication 4 comprenant en outre un agent de solubilisation de l'agent conservateur à une quantité égale ou voisine de 0,7 % en masse.

6. Lingette humide (10) composée d'un noyau formé de couches d'un matériau de support de la lingette humide (11, 12), où la lingette humide est imprégnée de la solution chimique pour lingette humide selon l'une quelconque des revendications 1 à 5.
